(19) Europäisches Patentamt

European Patent Office

Office européen des brevets

(11) Veröffentlichungsnummer: **0 442 430 B1**

(12) **EUROPÄISCHE PATENTSCHRIFT**

(45) Veröffentlichungstag der Patentschrift: **30.08.95**

(21) Anmeldenummer: **91101902.4**

(22) Anmeldetag: **11.02.91**

Die Akte enthält technische Angaben, die nach dem Eingang der Anmeldung eingereicht wurden und die nicht in dieser Patentschrift enthalten sind.

(51) Int. Cl.6: **C12P 7/40**, //(C12P7/40, C12R1:38),(C12P7/40, C12R1:40)

(54) **Mikrobiologische Oxidation von Methylgruppen in Heterocyclen.**

(30) Priorität: **13.02.90 CH 458/90**

(43) Veröffentlichungstag der Anmeldung:
**21.08.91 Patentblatt 91/34**

(45) Bekanntmachung des Hinweises auf die Patenterteilung:
**30.08.95 Patentblatt 95/35**

(84) Benannte Vertragsstaaten:
**AT BE CH DE DK ES FR GB IT LI LU NL SE**

(56) Entgegenhaltungen:
**EP-A- 0 233 656**
**US-A- 4 859 592**

**SOVIET INVENTIONS ILLUSTRATED, Ch Sektion, Woche Y-08, 05 April 1977, Derwent Publications LTD., London (GB); D 15, Seite 2**

**SOVIET INVENTIONS ILLUSTRATED, Ch Sektion, Band V, Nr. 44, 06 Dezember 1973, Derwent Publications LTD., London (GB); D 16 - E 14, Seite 4**

**SOVIET INVENTIONS ILLUSTRATED, Ch Sektion, Band W, Nr. 2, 18 Februar 1975, Derwent**

**Publications LTD., London (GB); D 16 - E 13**

**SOVIET INVENTIONS ILLUSTRATED, Ch Sektion, Mai 1969, Derwent Publications LTD., London (GB); Seite 3**

(73) Patentinhaber: **LONZA AG**
**(Geschäftsleitung: 4002 Basel)**
**CH-3945 Gampel/Wallis (CH)**

(72) Erfinder: **Kiener, Andreas, Dr.**
**Meisenweg 5**
**Visp (Kanton Wallis) (CH)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues mikrobiologisches Verfahren zur Oxidation von Methylgruppen in aromatischen 5- oder 6-Ring-Heterocyclen zur entsprechenden Carbonsäure, wobei der Heterocyclus keinen Substituenten am benachbarten Kohlenstoffatom der zu oxidierenden Methylgruppe aufweist. Dieser Heterocyclus dient als Substrat für die Umsetzung, die mittels Toluol-, Xylol- oder Cymol-verwertender Mikroorganismen der Gattung Pseudomonas durchgeführt wird, deren Enzyme zuvor induziert wurden.

Diese Heterocyclencarbonsäuren sind beispielsweise wichtige Zwischenprodukte zur Herstellung für Pharmazeutika. Beispielsweise ist Nikotinsäure (3-Pyridincarbonsäure) ein wichtiges Zwischenprodukt zur Herstellung von Nikotinsäureamid, welches ein Vitamin der B-Gruppe darstellt und eine wesentliche Bedeutung für die Ernährung von Mensch und Tier hat (Ullmann, Bd.19, 1980, S.603).

2-Pyrazincarbonsäure ist z.B. ein wichtiges Zwischenprodukt zur Herstellung des Tuberkulostatikums Pyrazinamid (2-Pyrazincarbonsäureamid) (Römpps Chemie Lexikon, Bd.5, 1987). 4-Thiazolcarbonsäure dient zur Herstellung von Thiabendazol, einem hochwirksamen Antihelmintikum (Wurmmittel), das wiederum Ausgangsmaterial für andere, neuere Antihelmintika, wie z.B. das Cambendazol, ist (Ullmann, Bd.23, 1980, S.46). 2-Thiophencarbonsäure besitzt antiallergische Wirkung (Ullmann, Bd.23, 1980, S.219).

Eingehende Untersuchungen zur Oxidation von Methylgruppen wurden bis jetzt mit aromatischen Kohlenwasserstoffen durchgeführt.

Die Produktion von Carbonsäuren durch mikrobielle Oxidation von methylierten Aromaten wurde ausführlich in den Arbeiten von Raymond und Mitarbeitern beschrieben (Raymond et al., Process Biochem., 1969, S.71-74).

Die US-PS 3 383 289 beschreibt ein Verfahren zur biochemischen Oxidation von Methylgruppen in aromatischen Kohlenwasserstoffen mit einem gram-positiven Mikroorganismenstamm der Gattung Nocardia.

Nachteile dieser Verfahren sind, dass beispielsweise bei der Methylgruppenoxidation von aromatischen Kohlenwasserstoffen der Benzolring der entsprechenden Säure gespalten wird.

In Pseudomonas putida ATCC 33015 ist bekannt, dass die biochemische Oxidation der Methylgruppe von Toluol in 3 Schritten zur Benzoesäure verläuft. Durch Einwirkung der Toluol-monoxygenase entsteht zuerst Benzylalkohol, welcher dann in zwei weiteren Schritten, katalysiert durch eine Alkohol- und eine Aldehyddehydrogenase, zur Säure überführt wird.

In diesem Stamm liegen sowohl die Xyl-Gene, die für Enzyme des Xylol-Abbaus kodieren, als auch die Gene, welche für die Regulation der Xyl-Gene verantwortlich sind, auf dem Plasmid pWWO. Dieses archaetypische Tol-Plasmid wurde molekularbiologisch bereits eingehend untersucht (Harayama et al., J. Bacteriol. 171, 1989, S. 5048-5055; Burlage et al., Appl. Environ Microbiol. 55, 1989, S. 1323-1328)

Die SU-PS 487 111 beschreibt ein Verfahren zur mikrobiologischen Oxidation von 2,6-Dimethylnaphthalin zu 6-Methyl-2-naphthalinsäure mit Hilfe von Mikroorganismen der Spezies Pseudomonas putida und Pseudomonas fluorescens. Das Endprodukt wird in einer Ausbeute von ungefähr 50% erhalten. Auch in der SU-PS 370 228 wird die mikrobiologische Oxidation von 2,6-Dimethylnaphthalin beschrieben, wobei die Umsetzungsbedingungen gezielt so gewählt werden, daß als Endprodukt 2,6-Naphthalindicarbonsäure erhalten wird.

Ebenso sind aus der Literatur mikrobiologische Verfahren zur Oxidation von Methylgruppen eines N-Heterocyclus bekannt. Nach der SU-PS 417 468 wird 2-Methylpyridin mit einem gram-positiven Mikroorganismenstamm der Gattung Nocardia zur entsprechenden Säure oxidiert.

Die SU-PS 228 688 beschreibt ein mikrobiologisches Verfahren zur Herstellung von Nikotinsäure aus 3-Methylpyridin mit einem gram-positiven Mikroorganismus der Gattung Mycobakterium. Aus der SU-PS 302 341 ist ein mikrobiologisches Verfahren zur Herstellung von Nikotinsäure mit gram-positiven Bakterien der Gattung Nocardia bekannt.

Die Nachteile der Methylgruppenoxidation von N-Heterocyclen mit gram-positiven Bakterien bestehen darin, dass bei diesen Alkan-verwertenden Bakterien das Mischungsverhältnis des Alkans zur oxidierenden Substanz genau abgestimmt sein muss, um eine Biotransformation zu erreichen, und dass keine Biotransformation des Substrates in Abwesenheit des Alkans stattfindet, d.h., das zur Induktion verwendete Alkan muss immer, auch bei der Umsetzung des Substrates, anwesend sein. Durch Vergleichsversuche mit dem gram-positiven Bakterium Nocardia und unserem gram-negativen Pseudomonas konnte eindeutig gezeigt werden, dass Nocardia sogar in Gegenwart eines Alkans, wie z.B. Dodecan, 3-Methylpyridin nicht zur Nikotinsäure oxidiert.

Des weiteren beschreibt die US-PS 4 859 592 ein Verfahren zur Herstellung von Picolinsäure mit Pseudomonas putida, indem ein Alkyl-substituierter aromatischer Kohlenwasserstoff in Gegenwart von molekularem Sauerstoff in einer ersten Stufe durch eine Dioxygenase ein 2-Hydroxymuconsäuresemialdehyd gebildet wird, dieser dann in einer zweiten Stufe mit Ammoniak oder einem primären Amin zur 2-Picolinsäure umgesetzt wird.

Der Nachteil dieses Verfahrens besteht darin, dass die entsprechende Picolinsäure erst in der zweiten Stufe durch die Umsetzung des 2-Hydroxymuconsäuresemialdehyds mit Ammoniak gebildet wird.

Die Aufgabe der vorliegenden Erfindung bestand darin, diese Nachteile auszuschalten und ein einfaches und einstufiges Verfahren zur mikrobiologischen Methylgruppenoxidation zu entwickeln mit dem die entsprechenden Säuren in guter Ausbeute und Reinheit isoliert werden können und der aromatische Heterocyclus nicht gespalten wird.

Eine weitere Aufgabe bestand darin, ein Verfahren zur Verfügung zu stellen, bei dem die zur Induktion verwendete Verbindung, nach erfolgter Induktion der Enzyme, während der Umsetzung des Substrates nicht mehr anwesend sein muss und damit die Umsetzung nicht von der Menge des Enzyminduktors abhängt.

Diese Aufgabe wurde gemäss Patentanspruch 1 gelöst.

Gegenstand der Erfindung ist ein mikrobiologisches Verfahren zur Oxidation von Methylgruppen in aromatischen 5- oder 6-Ring-Heterocyclen, wobei der Heterocyclus keinen Substituenten am benachbarten Kohlenstoffatom der zu oxidierenden Methylgruppe aufweist, zur entsprechenden Carbonsäure, wobei der methylierte Heterocyclus als Substrat für die Umsetzung eingesetzt wird und die Umsetzung mittels Toluol-, Xylol- oder Cymol-verwertender Mikroorganismen der Gattung Pseudomonas durchgeführt wird, deren Enzyme zuvor induziert wurden.

Zweckmässig wird die Umsetzung mittels Toluol-, Xylol- oder Cymol-verwertender Mikroorganismen der Spezies Pseudomonas putida durchgeführt.

Vorzugsweise wird der Xylol-verwertende Mikroorganismenstamm Pseudomonas putida mit der Bezeichnung ATCC 33015 oder eine wirksame Mutante von diesem oder der Cymol-verwertende Mikroorganismenstamm Pseudomonas putida mit der Bezeichnung DSM 5709 oder eine wirksame Mutante von diesem, angewendet. Insbesondere wird die Umsetzung mit dem Mikroorganismenstamm Pseudomonas putida ATCC 33105 durchgeführt.

Der Mikroorganismenstamm Pseudomonas putida (DSM 5709) wurde bei der Deutschen Sammlung von Mikroorganismen (DSM) und Zellkulturen GmbH, Mascheroderweg 1b, 3300 Braunschweig, BRD, am 22.12.1989 unter der Nummer DSM 5709 hinterlegt.

Der Mikroorganismenstamm Pseudomonas putida mit der Bezeichnung ATCC 33015 ist bei der American Type Culture Collection 12301 Parklawn Drive Rockville, Maryland 20852, USA, unter der Nummer ATCC 33015 hinterlegt.

Die Enzyminduktion kann zweckmässig sowohl mit Verbindungen durchgeführt werden, die dem Mikroorganismus als Kohlenstoff- und Energiequelle dienen, wie beispielsweise p-Xylol, m-Xylol, p-Cymol, m-Cymol und Toluol, als auch mit Verbindungen, die dem Mikroorganismus nicht als Kohlenstoff- und Energiequelle dienen, wie beispielsweise mono- und disubstituierte Methyl-, Ethyl- und Chlortoluole, Benzylalkohole und p-Chlorbenzaldehyd, die schon als Enzyminduktoren für den Abbau aromatischer Kohlenwasserstoffe beschrieben sind (Abril M.-A. et al., J.Bacteriol., Vol. 171, 1989, S.6782-6789). Vorzugsweise wird die Enzyminduktion mit p-Xylol, m-Xylol, 2-Chlortoluol oder 2-Bromtoluol durchgeführt.

Die zur Induktion verwendeten Verbindungen können entweder während der Umsetzung des Substrats anwesend sein oder deren Zufuhr kann vor der Umsetzung des Substrats unterbunden werden. Die Induktorkonzentration wird üblicherweise so gewählt, dass sie tiefer als die minimale Hemmkonzentration der für die Umsetzung verantwortlichen Enzyme liegt.

Vorzugsweise wird je nach Ausführungsform des Verfahrens die Zufuhr der zur Induktion verwendeten Verbindungen während der Umsetzung des Substrates entweder durch Stoppen der Zufuhr oder durch Abzentrifugieren der Zellen unterbunden.

Die genannten Stämme wachsen üblicherweise mit p-Xylol, m-Xylol, p-Cymol, m-Cymol oder mit Toluol als einzige Kohlenstoff- und Energiequelle in einem Mineralmedium (Kulla et al., Arch. Microbiol 135, 1983, S.1-7) oder in einem Komplexmedium ("Nutrient Broth Nr.2" Oxoid Ltd., GB) oder in einem Minimalmedium, dessen Zusammensetzung in Tabelle 3 angegeben ist. Das Wachstumsubstrat wurde gemäss den Angaben von Claus und Walker (J. Gen. Microbiol. 36, 1964, S.107-122) gasförmig dem Medium zugeführt, wobei die Begasungsrate 0,5 V/min betrug.

Vor der Substratzugabe werden die Zellen bis zu einer optischen Dichte von 1 bis 200 bei 650 nm im Kulturmedium angezogen, vorzugsweise bis zu einer optischen Dichte von 5 bis 100 bei 650 nm.

Die Umsetzung kann zweckmässig entweder unter einmaliger oder kontinuierlicher Substratzugabe erfolgen, so dass die Substratkonzentration im Kulturmedium 20% (w/v) nicht übersteigt. Vorzugsweise erfolgt die Substratzugabe so, dass die Substratkonzentration im Kulturmedium 5% (w/v) nicht übersteigt. Die Substratzugabe kann auch, je nach Ausführungsform des Verfahrens, gleichzeitig mit dem Enzyminduktor erfolgen, beispielsweise indem ein Gemisch von Enzyminduktor und Substrat eingesetzt wird.

Die Umsetzung wird zweckmässig in einem pH-Bereich von 4 bis 11, vorzugsweise von 6 bis 10 durchgeführt.

Zweckmässig wird die Umsetzung üblicherweise bei einer Temperatur von 15 bis 50°C, vorzugsweise bei einer Temperatur von 25 bis 40°C durchgeführt.

Die Umsetzung wird üblicherweise in einer Zeit von 1 bis 24 Stunden durchgeführt.

Als Substrate können für die Umsetzung zweckmässig methylierte aromatische 5-Ring-Heterocyclen verwendet werden, die ein oder mehrere Heteroatome aus der Reihe Sauerstoff, Stickstoff, Schwefel enthalten, wie beispielsweise methylierte Thiophene, Furane, Pyrrole, Thiazole, Pyrazole oder Imidazole, die keinen Substituenten am benachbarten Kohlenstoffatom der zu oxidierenden Methylgruppe aufweisen; vorzugsweise werden Furane, Thiophene, Pyrrole und Thiazole angewendet. Insbesondere werden als 5-Ring-Heterocyclen 3,5-Dimethylpyrazol, 5-Methylthiazol, 4-Methylthiazol, 2,5-Dimethylthiophen, 2-Methylthiophen, 3-Methylthiophen, 2,5-Dimethylfuran und 2,5-Dimethylpyrrol angewendet .

Zweckmässig kann die Umsetzung mit aromatischen methylierten 6-Ring-Heterocyclen mit ein oder mehreren Stickstoffatomen als Heteroatom durchgeführt werden, wie beispielsweise methylierte Pyridine, Pyrimidine, Pyrazine oder Pyridazine, die keinen Substituenten am benachbarten Kohlenstoffatom der zu oxidierenden Methylgruppe aufweisen, vorzugsweise werden Pyridine, Pyrazine und Pyrimidine wie beispielsweise 2-Methylpyridin, 3-Methylpyridin, 4-Methylpyridin, 2,5-Dimethylpyridin, 2,4-Dimethylpyridin, 6-Chlor-3-methylpyridin, 2-Chlor-3-ethyl-6-methylpyridin, 4,6-Dimethylpyrimidin, 2-Methylpyrazin, 2,5-Dimethylpyrazin, 2,6-Dimethylpyrazin, 2,3,5-Trimethylpyrazin und 2-Chlor-3,6-dimethylpyrazin angewendet.

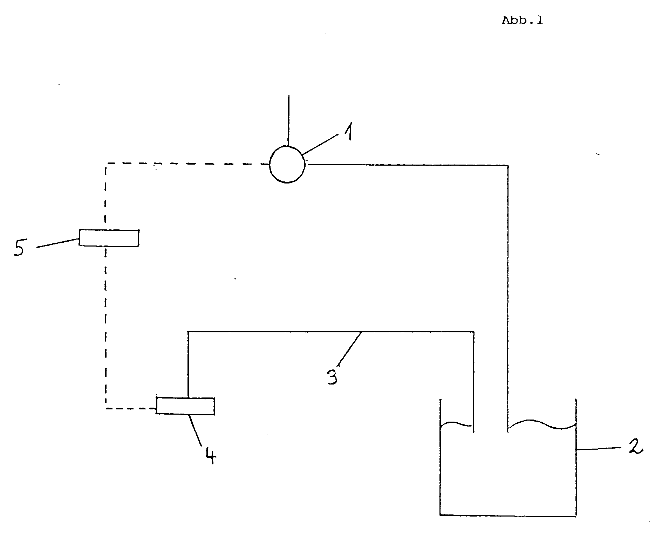

Eine vorzugsweise Ausführungsform des Verfahrens ist in Abb. 1 dargestellt.

Nach dieser Ausführungsform kann der Enzyminduktor und/oder der methylierte Heterocyclus als Substrat in einen Bioreaktor (2) zugeführt werden, wobei die Zufuhrmenge über die Konzentration des Enzyminduktors in der Abluft (3) des Bioreaktors (2) geregelt wird.

Vorzugsweise wird die Konzentration des Enzyminduktors in der Abluft (3) mit einer Messvorrichtung (4) gemessen.

Als Messvorrichtung für die Enzyminduktor-Konzentration kann beispielsweise ein Photometer für ultraviolettes Licht, ein Gaschromatograph oder ein Gaschromatograph gekoppelt mit einem Massenspektrometer dienen.

Diese Messvorrichtung (4) ist zweckmässig über eine Steuerung (5), die die Zufuhrmenge des Enzyminduktors und/oder des methylierten Heterocyclus regelt mit einer Pumpe (1), über die die Zufuhr des Enzyminduktors und/oder des methylierten Heterocyclus in den Bioreaktor (2) erfolgt, gekoppelt.

Durch diese Kopplung wird zweckmässig die Zufuhr des Enzyminduktors und/oder des methylierten Heterocyclus geregelt. Vorzugsweise wird durch diese Regelung die Konzentration des Enzyminduktors in der Abluft (3) konstant gehalten.

Zweckmässig erfolgt die Regelung so, dass die Konzentration des Enzyminduktors in der Abluft (3) zwischen 0,001 mMol/l Abluft und 10 mMol/l Abluft , vorzugsweise zwischen 0,01 mMol/l Abluft und 3 mMol/l Abluft liegt.

Vorzugsweise wird der Enzyminduktor und/oder der methylierte Heterocyclus als Substrat in Form einer Mischung in den Bioreaktor (2) zugeführt.

Das Verhältnis von Enzyminduktor zu Substrat liegt zweckmässig zwischen 5:1 und 3:1.

Zweckmässig wird nach dieser Ausführungsform der Enzyminduktor und/oder der methylierte Heterocyclus über die Zuluft dem Bioreaktor (2) zugeführt.

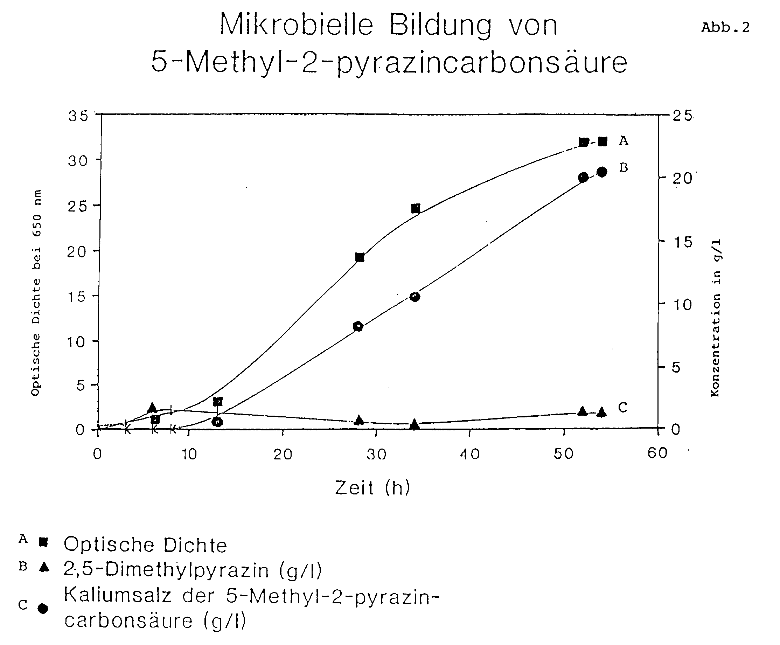

Die Abb. 2 zeigt die Resultate der Biotransformation in der vorzugsweisen Ausführungsform.

Nach der Umsetzung können die entsprechenden Säuren auf bekannte Art und Weise isoliert werden, beispielsweise durch Extraktion mit organischen Lösungsmitteln oder, falls das Alkalisalz der Heterocycluscarbonsäure gebildet wird, durch Einengen des zellfreien Kulturmediums.

Das Alkalisalz der Heterocycluscarbonsäure kann beispielsweise durch Zugabe eines Alkalihydroxids zur pH-Wert-Einstellung des Kulturmediums gebildet werden.

Erfindungsgemäss wird ein einfaches und einstufiges mikrobiologisches Verfahren zur Oxidation von Methylgruppen in aromatischen 5- oder 6-Ring-Heterocyclen zur Verfügung gestellt, mit dem die entsprechenden Säuren in guter Ausbeute und Reinheit isoliert werden.

Ein weiterer Vorteil dieses Verfahrens besteht darin, dass der aromatische Heterocyclus nicht gespalten wird und die Umsetzungsrate nicht von der Menge des Enzyminduktors abhängt.

4

Beispiel 1

5-Methyl-2-pyrazincarbonsäure

Pseudomonas putida ATCC 33015 wurde in einem Komplexmedium (100 ml) "Nutrient Broth Nr. 2" (Oxoid Ltd., England) in einem Fermenter bei pH 7,0 und einer Temperatur von 30°C angezogen, wobei der Enzyminduktor p-Xylol gasförmig gemäss den Angaben von Claus und Walker (J. Gen. Microbiol. 36, 1964, S.107-122) bis zu einer Konzentration von 1 mMol/Liter zugeführt wurde.
Anschliessend wurden die Zellen zweimal mit Mineralmedium (Kulla et al., Arch. Microbiol. 135, 1983, S.1-7) gewaschen und eine optische Dichte von 10 bei 650 nm in 100 ml Mineralmedium eingestellt. Zu dieser Zellsuspension wurde 1 mMol 2,5-Dimethylpyrazin hinzugegeben, was einer Substratkonzentration von 0,108% (w/v) in 100 ml entspricht. Nach einer Inkubation von 4 Stunden bei 30°C wurden in Abwesenheit vom Enzyminduktor 0,9 mMol 5-Methyl-2-pyrazincarbonsäure entsprechend einer Ausbeute von 90%, bezogen auf eingesetztes 2,5-Dimethylpyrazin, erhalten.

Beispiel 2

Entsprechend zu Beispiel 1 wurden auch folgende Verbindungen als Enzyminduktor angewendet, die mit der entsprechenden Konzentration in Tabelle 1 zusammengefasst sind.

Tabelle 1

| Enzyminduktor | Konzentr. [Mole in 100 ml Zellen] | Substratmenge [1 mMol in 100 ml Zellen entspr. 0,108% (w/v)] | % Ausbeute an 5-Methyl-2-pyrazin-carbonsäure |
|---|---|---|---|
| o-Xylol | 0,1 mMol | 2,5-Dimethylpyrazin | 90 |
| m-Xylol | 0,1 mMol | 2,5-Dimethylpyrazin | 90 |
| 2-Chlortoluol | 0,1 mMol | 2,5-Dimethylpyrazin | 90 |
| 2-Bromtoluol | 0,1 mMol | 2,5-Dimethylpyrazin | 90 |

Beispiel 3

5-Methyl-2-pyrazincarbonsäure

Pseudomonas putida ATCC 33015 wurde entsprechend zu Bsp. 1 jedoch in Mineralmedium (Kulla et al., Arch. Microbiol. 135, 1983, S.1-7) mit p-Xylol als einziger Kohlenstoff- und Energiequelle angezogen. Zur Zellsuspension (100 ml) mit einer opt. Dichte von 10 wurde 1 mMol 2,5-Dimethylpyrazin, was einer Konzentration von 0,108% (w/v) entspricht, hinzugegeben. Während der Umsetzung des Substrates wurde die p-Xylol-Zugabe unterbunden. Unter diesen Bedingungen wurde 1 mMol 2,5-Dimethylpyrazin in einem Zeitraum von 4 Stunden zu 0,9 mMol 5-Methyl-2-pyrazincarbonsäure, entsprechend einer Ausbeute von 90%, bez. auf einges. 2,5-Dimethylpyrazin, umgesetzt.

Beispiel 4

Pseudomonas putida DSM 5709 wurde analog zu Beispiel 3, jedoch mit p-Cymol als einziger Kohlenstoff- und Energiequelle, angezogen. Nach einem Zeitraum von 16 Stunden wurden 0,5 mMol 5-Methyl-2-pyrazincarbonsäure, entsprechend einer Ausbeute von 50%, bez. auf einges. 2,5-Dimethylpyrazin, erhalten.
Die Beispiele 5 bis 28 wurden entsprechend zu Beispiel 3 mit einer Menge von 1 mMol Substrat pro 100 ml Zellsuspension durchgeführt und sind in Tabelle 2 zusammengefasst.

T a b e l l e   2

| Beispiel | Substrat | Konzentration des Substr. in % (w/v) im Kulturmedium | Reaktionszeit in Stunden | Endprodukt | Ausbeute in % |
|---|---|---|---|---|---|
| 5 | 2-Methylpyrazin | 0,049 | 16 | 2-Pyrazincarbonsäure | 30 |
| 6 | 2,5-Dimethyl-pyrazin | 0,108 | 4 | 2-Methyl-5-pyrazincarbonsäure | 90 |
| 7 | 2,6-Dimethyl-pyrazin | 0,108 | 4 | 2-Methyl-6-pyrazincarbonsäure | 90 |
| 8 | 2,3,5-Trimethylpyrazin | 0,122 | 16 | 2,3-Dimethyl-5-pyrazincarbonsäure | 50 |
| 9 | 2-Chlor-3,6-dimethylpyrazin | 0,142 | 16 | 2-Chlor-3-methyl-6-pyrazincarbonsäure | 90 |
| 10 | 2-Methylpyridin | 0,093 | 16 | 2-Pyridincarbonsäure | 90 |
| 11 | 3-Methylpyridin | 0,093 | 16 | 3-Pyridincarbonsäure | 50 |
| 12 | 4-Methylpyridin | 0,093 | 16 | 4-Pyridincarbonsäure | 30 |

EP 0 442 430 B1

| Beispiel | Substrat | Konzentra-tion des Substr. in % (w/v) im Kulturmedium | Reaktions-zeit in Stunden | Endprodukt | Ausbeute in % |
|---|---|---|---|---|---|
| 13 | 2,6-Dimethylpyridin | 0,107 | 16 | 6-Methyl-2-pyridin-carbonsäure | 80 |
| 14 | 2,5-Dimethylpyridin | 0,107 | 16 | 5-Methyl-2-pyridin-carbonsäure | 40 |
| 15 | 2,4-Dimethylpyridin | 0,107 | 16 | 4-Methyl-2-pyridin-carbonsäure | 40 |
| 16 | 3,5-Dimethylpyridin | 0,107 | 16 | 5-Methyl-3-pyridin-carbonsäure | 40 |
| 17 | 6-Chlor-2-methylpyridin | 0,128 | 16 | 6-Chlor-2-pyridin-carbonsäure | 90 |
| 18 | 6-Chlor-3-methylpyridin | 0,128 | 16 | 6-Chlor-3-pyridin-carbonsäure | 90 |
| 19 | 2-Chlor-3-ethyl-6-methyl-pyridin | 0,157 | 16 | 2-Chlor-3-ethyl-6-pyridincarbonsäure | 10 |

| | | | | |
|---|---|---|---|---|
| 20 | 4,6-Dimethylpyrimidin | 0,108 | 16 | 6-Methyl-4-pyrimi-dincarbonsäure | 20 |
| 21 | 3,5-Dimethylpyrazol | 0,096 | 16 | 5-Methyl-3-pyrazol-carbonsäure | 80 |
| 22 | 5-Methylthiazol | 0,099 | 16 | 5-Thiazolcarbonsäure | 80 |
| 23 | 4-Methylthiazol | 0,099 | 16 | 4-Thiazolcarbonsäure | 80 |
| 24 | 2,5-Dimethylthiophen | 0,112 | 16 | 5-Methyl-2-thio-phencarbonsäure | 90 |
| 25 | 2-Methylthiophen | 0,098 | 16 | 2-Thiophencarbonsäure | 90 |
| 26 | 3-Methylthiophen | 0,098 | 16 | 3-Thiophencarbonsäure säure | 90 |
| 27 | 2,5-Dimethylfuran | 0,096 | 16 | 5-Methyl-2-furan-carbonsäure | 40 |
| 28 | 2,5-Dimethylpyrrol | 0,095 | 16 | 5-Methyl-2-pyrrol-carbonsäure | 40 |

EP 0 442 430 B1

EP 0 442 430 B1

Beispiel 29

Produktion von 5-Methyl-2-pyrazincarbonsäure

Pseudomonas putida ATCC 33015 wurde einem Minimalmedium, dessen Zusammensetzung in der Tabelle 3 angegeben ist, in einem 20 l Bioreaktor (2) bei einem Arbeitsvolumen von 15 l angezogen.

Die Temperatur betrug 30°C; der pH-Wert wurde durch Dosierung von Kaliumhydroxid auf einen Wert von 7,0 konstant gehalten. Die Begasungsrate betrug 20 l pro Minute. Als Wachstumssubstrat diente ein Gemisch aus 4 Teilen (v/v) p-Xylol und 1 Teil 2,5-Dimethylpyrazin. Die Zufuhr dieser Mischung erfolgte über die Pumpe (1) in den Bioreaktor (2). Die Dosierung des Wachstumssubstrates wurde über eine Xylol-Messung (4) in der Bioreaktor-Abluft (3) mit einer Steuerung (5) gekoppelt. Dabei wurde durch die Steuerung (5) die Xylol-Konzentration in der Abluft (3) auf einen Wert von 0,2 mMol/l konstant gehalten. (Abb. 1)

Die Biotransformation wurde erst dann abgebrochen, wenn kein Wachstum mehr feststellbar war. Abb. 2 zeigt die Resultate einer solchen Biotransformation.

Die Kurve A der Abb. 2 stellt die optische Dichte bei 650 nm dar.

Die Kurve B der Abb. 2 stellt die Konzentration in g/l von 2,5-Dimethylpyrazin dar.

Die Kurve C der Abb. 2 stellt die Konzentration in g/l von Kaliumsalz der 5-Methyl-2-pyrazincarbonsaure dar.

Mit dieser Technik wurden 5-Methyl-2-pyrazincarbonsäure-Konzentrationen von 38 g/l erhalten.

Tabelle 3

| Mediumzusammensetzung: | |
|---|---|
| - Mg Cl$_2$ • 6H$_2$O | 0,8 g/l |
| - Ca Cl$_2$ | 0,16 g/l |
| - (NH$_4$)SO$_4$ | 2 g/l |
| - NH$_4$Cl | 5 g/l |
| - Na$_2$SO$_4$ | 0,25 g/l |
| - KH$_2$SO$_4$ | 0,4 g/l |
| - Na$_2$HPO$_4$ | 0,9 g/l |
| - Spurenelemente | 1 ml/l |
| - FeEDTA | 15 ml/l |
| Zusammensetzung der Spurenelementlösung: | |
| - KOH | 15 g/l |
| - EDTANa$_2$ • 2H$_2$O | 10 g/l |
| - ZnSO$_4$ • 7H$_2$O | 9 g/l |
| - MnCl$_2$ • 4H$_2$O | 4 g/l |
| - H$_3$BO$_3$ | 2,7 g/l |
| - CoCl$_2$ • 6H$_2$O | 1,8 g/l |
| - CuCl$_2$ • 2H$_2$O | 1,5 g/l |
| - NiCl$_2$ • 6H$_2$O | 0,18 g/l |
| - Na$_2$MoO$_4$ • 2H$_2$O | 0,2 g/l |
| Zusammensetzung von FeEDTA: | |
| - EDTA Na$_2$ • 2H$_2$O | 5 g/l |
| - FeSO$_4$ • 7H$_2$O | 2 g/l |

Vergleichsbeispiele

a) Pseudomonas putida ATCC 33015

Die Biotransformation von 3-Methylpyridin mit Pseudomonas putida ATCC 33015 wurde gemäss Beispiel 3 mit 1 mMol 3-Methylpyridin durchgeführt. Nach einer Inkubationszeit von 8 Stunden wurden 0,25 mMol Nikotinsäure entsprechend einer Ausbeute von 25%, bez. auf einges. 3-Methylpyridin, erhalten.

9

b) Rhodoccocus rhodochrous bzw. Nocardia

Rhodoccocus rhodochrous DSM 43002 (ATCC 19149) wurde im Mineralmedium gemäss Beispiel 3 mit 0,4% Dodecan als Kohlenstoff- und Energiequelle angezogen und anschliessend im gleichen Mineralmedium, jedoch ohne Dodecan, gewaschen. Die Zellsuspensionen (100 ml) mit einer optischen Dichte bei 650 nm von 10 wurden in drei getrennten Kolben mit folgenden Verbindungen versetzt:

a) 1 mMol 3-Methylpyridin, b) 1 mMol 3-Methylpyridin und 0,055 mMol Dodecan, c) 1 mMol 3-Methylpyridin und 5,5 mMol Dodecan.

Nach 8 Stunden Inkubationszeit bei 30°C konnte in keinem der Ansätze Nikotinsäure nachgewiesen werden.

**Patentansprüche**

1. Mikrobiologisches Verfahren zur Oxidation von Methylgruppen in aromatischen 5- oder 6-Ring-Heterocyclen, wobei der Heterocyclus keinen Substituenten am benachbarten Kohlenstoffatom der zu oxidierenden Methylgruppe aufweist, zur entsprechenden Carbonsäure, dadurch gekennzeichnet, dass man den methylierten Heterocyclus als Substrat für die Umsetzung einsetzt und die Umsetzung mittels Toluol-, Xylol- oder Cymol-verwertender Mikroorganismen der Gattung Pseudomonas durchgeführt wird, deren Enzyme zuvor induziert wurden.

2. Verfahren nach Patentanspruch 1, dadurch gekennzeichnet, dass die Enzyminduktion entweder mit Verbindungen durchgeführt wird, die dem Mikroorganismus als Kohlenstoff- und Energiequelle dienen, oder mit Verbindungen, die dem Mikroorganismus nicht als Kohlenstoff- und Energiequelle dienen.

3. Verfahren nach mindestens einem der Patentansprüche 1 bis 2, dadurch gekennzeichnet, daß die Umsetzung mittels Toluol-, Xylol- oder Cymol-verwertender Mikroorganismen der Spezies Pseudomonas putida durchgeführt wird.

4. Verfahren nach mindestens einem der Patentansprüche 1 bis 3, dadurch gekennzeichnet, daß die Umsetzung mit dem Xylol-verwertenden Mikroorganismenstamm Pseudomonas putida mit der Bezeichnung ATCC 33015 oder einer wirksamen Mutante von diesem durchgeführt wird.

5. Verfahren nach mindestens einem der Patentansprüche 1 bis 3, dadurch gekennzeichnet, daß die Umsetzung mit dem Cymol-verwertenden Mikroorganismenstamm Pseudomonas putida mit der Bezeichnung DSM 5709 oder einer wirksamen Mutante von diesem durchgeführt wird.

6. Verfahren nach mindestens einem der Patentansprüche 1 bis 5, dadurch gekennzeichnet, daß die Umsetzung entweder unter einmaliger oder unter kontinuierlicher Substratzugabe durchgeführt wird, so daß die Substratkonzentration im Kulturmedium 20% (w/v) nicht übersteigt.

7. Verfahren nach mindestens einem der Patentansprüche 1 bis 6, dadurch gekennzeichnet, daß die Umsetzung bei einem pH von 4 bis 11 durchgeführt wird.

8. Verfahren nach mindestens einem der Patentansprüche 1 bis 7, dadurch gekennzeichnet, daß man die Umsetzung bei einer Temperatur von 15 bis 50°C durchführt.

9. Verfahren nach mindestens einem der Patentansprüche 1 bis 8, dadurch gekennzeichnet, daß man die Umsetzung mit einem methylierten aromatischen 5-Ring-Heterocyclus als Substrat durchführt, der ein oder mehrere Heteroatome aus der Reihe Sauerstoff, Stickstoff, Schwefel enthält, z.B. mit einem methylierten Thiophen, Furan, Pyrrol, Thiazol, Pyrazol oder mit einem Imidazol.

10. Verfahren nach mindestens einem der Patentansprüche 1 bis 8, dadurch gekennzeichnet, daß man die Umsetzung mit einem methylierten 6-Ring-Heterocyclus als Substrat durchführt, der ein oder mehrere Stickstoffatome als Heteroatom enthält, z.B. mit einem methylierten Pyridin, Pyrimidin, Pyrazin oder mit einem Pyridazin.

11. Verfahren nach mindestens einem der Patentansprüche 1 bis 10, dadurch gekennzeichnet, daß man den Enzyminduktor und/oder den methylierten Heterocyclus als Substrat in einen Bioreaktor (2) zuführt, wobei die Zufuhrmenge über die Konzentration des Enzyminduktors in der Abluft (3) des Bioreaktors

EP 0 442 430 B1

(2) geregelt wird.

12. Verfahren nach Patentanspruch 11, dadurch gekennzeichnet, daß die Konzentration des Enzyminduktors in der Abluft (3) mit einer Messvorrichtung (4) gemessen wird und die Zufuhrmenge des Enzyminduktors und/oder des methylierten Heterocyclus über eine Steuerung (5), die an die Messvorrichtung (4) gekoppelt ist, geregelt wird.

13. Verfahren nach mindestens einem der Patentansprüche 11 bis 12, dadurch gekennzeichnet, daß die Regelung so erfolgt, daß die Konzentration des Enzyminduktors in der Abluft (3) konstant gehalten wird und zwischen 0,001 mMol/l Abluft und 10 mMol/l Abluft liegt.

**Claims**

1. A microbiological process for oxydizing methyl groups in 5- or 6-atomic heterocyclic compounds to the corresponding carboxylic acid, the heterocyclic compound having no substituent on the carbon atom adjacent to the methyl group to be oxydized, characterized in that the methylated heterocyclic compound is used as substrate for the conversion and conversion is carried out by means of toluene-, xylene- or cymene-utilizing microorganisms of the genus Pseudomonas, the enzymes of which have been induced previously.

2. Process according to claim 1, characterized in that the enzyme induction is carried out either using compounds which serve the microorganism as source of carbon and energy or with compounds which do not serve the microorganism as source of carbon and energy.

3. Process according to at least one of claims 1 to 2, characterized in that the conversion is carried out by means of toluene-, xylene- or cymene-utilizing microorganisms of the species Pseudomonas putida.

4. Process according to at least one of claims 1 to 3, characterized in that the conversion is carried out with the xylene-utilizing microorganism strain Pseudomonas putida having the designation ATCC 33015, or with an active mutant thereof.

5. Process according to at least one of claims 1 to 3, characterized in that the conversion is carried out with the cymene-utilizing microorganism strain Pseudomonas putida having the designation DSM 5709, or with an active mutant thereof.

6. Process according to at least one of claims 1 to 5, characterized in that the conversion is carried out by adding the substrate either once or continuously so that the substrate concentration in the culture medium does not exceed 20% (w/v).

7. Process according to at least one of claims 1 to 6, characterized in that the conversion is carried out at a pH of from 4 to 11.

8. Process according to at least one of claims 1 to 7, characterized in that the conversion is carried out at a temperature of from 15 to 50°C.

9. Process according to at least one of claims 1 to 8, characterized in that the conversion is carried out with a methylated 5-ring heterocylic compound as substrate, which contains one or more heteroatoms of the group of oxygen, nitrogen, sulphur, e.g. with a methylated thiophene, furane, pyrrole, thiazole, pyrazole or with an imidazole.

10. Process according to at least one of claims 1 to 8, characterized in that the conversion is carried out with a methylated 6-ring heterocylic compound as substrate, which contains one or more nitrogen atoms as heteroatoms, e.g. with a methylated pyridine, pyrimidine, pyrazine or with a pyridazine.

11. Process according to at least one of claims 1 to 10, characterized in that the enzyme inductor and/or the methylated heterocylic compound is fed as substrate into a bioreactor (2), the feed rate being regulated through the concentration of the enzyme inductor in the outgoing air (3) of the bioreactor (2).

11

**12.** Process according to claim 11, characterized in that the concentration of the enzyme inductor in the outgoing air (3) is determined using a measuring apparatus (4) and the feed rate of the enzyme inductor and/or of the heterocyclic compound is regulated by a controlling means (5) which is coupled to the measuring apparatus (4).

**13.** Process according to at least one of claims 11 to 12, characterized in that the regulation is effected in such a manner that the concentration of the enzyme inductor in the outgoing air (3) is kept constant and is between 0.001 mmole/l outgoing air and 10 mmole/l outgoing air.

**Revendications**

**1.** Procédé microbiologique pour l'oxydation de groupes méthyle dans des hétérocycles aromatiques à 5 ou 6 éléments en l'acide carboxylique correspondant, l'hétérocycle ne présentant aucun substituant sur l'atome de carbone voisin du groupe méthyle à oxyder, caractérisé en ce que l'on utilise comme substrat pour la réaction, l'hétérocycle méthylé et l'on conduit la réaction au moyen de microorganismes utilisant le toluol, le xylol ou le cymol, microorganismes de l'espèce Pseudomonas, dont les enzymes ont été préalablement induites.

**2.** Procédé selon la revendication 1, caractérisé en ce que l'induction enzymatique est conduite soit avec des composés qui servent de sources de carbone et d'énergie aux microorganismes soit avec des composés qui ne servent pas de sources de carbone et d'énergie aux microorganismes.

**3.** Procédé selon au moins l'une des revendications 1 à 2, caractérisé en ce que la réaction est conduite au moyen de microorganismes utilisant le toluol, le xylol ou le cymol, microorganismes de l'espèce Pseudomonas putida.

**4.** Procédé selon au moins l'une des revendications 1 à 3, caractérisé en ce que la réaction est conduite avec la souche de microorganisme Pseudomonas putida utilisant le xylol portant la désignation ATCC 33015 ou avec un mutant actif de cette souche.

**5.** Procédé selon au moins l'une des revendications 1 à 3, caractérisé en ce que la réaction est conduite avec la souche de microorganisme Pseudomonas putida utilisant le cymol portant la désignation DSM 5709 ou un mutant actif de cette souche.

**6.** Procédé selon au moins l'une des revendications 1 à 5, caractérisé en ce que la réaction est conduite soit sous addition en une seule fois soit en continu du substrat de façon que la concentration de substrat dans le milieu de culture ne dépasse pas 20 % en poids.

**7.** Procédé selon au moins l'une des revendications 1 à 6, caractérisé en ce que la réaction est conduite avec un pH de 4 à 11.

**8.** Procédé selon au moins l'une des revendications 1 à 7, caractérisé en ce que la réaction est conduite à une température de 15 à 50°C.

**9.** Procédé selon au moins l'une des revendications 1 à 8, caractérisé en ce que la réaction est conduite avec un hétérocycle méthylé aromatique à 5 éléments comme substrat, qui contient un ou plusieurs hétéro-atomes de la série oxygène, azote, soufre, par exemple avec un thiophène méthylé, furanne, pyrrole, thiazole, pyrazole ou avec un imidazole.

**10.** Procédé selon au moins l'une des revendications 1 à 8, caractérisé en ce que la réaction est conduite avec un hétérocycle méthylé à 6 membres comme substrat, qui contient un ou plusieurs atomes d'azote comme hétéro-atomes, par exemple avec une pyridine, pyrimidine, pyrazine ou pyridazine méthylée.

**11.** Procédé selon au moins l'une des revendications 1 à 10, caractérisé en ce que l'on ajoute l'inducteur enzymatique et/ou l'hétérocycle méthylé comme substrat dans un bioréacteur (2), la quantité d'addition étant régulée par la concentration de l'inducteur enzymatique dans la sortie d'air (3) du bioréacteur (2).

**EP 0 442 430 B1**

12. Procédé selon la revendication 11, caractérisé en ce que la concentration de l'inducteur enzymatique est mesurée dans l'air de sortie (3) avec un dispositif de mesure (4) et le volume d'amenée de l'inducteur enzymatique et/ou de l'hétérocycle méthylé est régulé par une commande (5) qui est accouplée au dispositif de mesure (4).

13. Procédé selon au moins l'une des revendications 11 à 12, caractérisé en ce que la régulation s'effectue de manière à maintenir constante la concentration de l'inducteur enzymatique dans l'air de sortie (3) dans une fourchette entre 0,001 mmole/l et 10 mmoles/l d'air de sortie.

Abb.1

# Mikrobielle Bildung von 5-Methyl-2-pyrazincarbonsäure

Abb.2

A ■ Optische Dichte
B ▲ 2,5-Dimethylpyrazin (g/l)
C ● Kaliumsalz der 5-Methyl-2-pyrazin-carbonsäure (g/l)